# EUROPEAN PATENT SPECIFICATION

(11) **EP 2 823 313 B1**
(45) Date of publication and mention of the grant of the patent: **18.09.2019**
(21) Application number: 13710342.0
(22) Date of filing: 08.03.2013
(51) Int. Cl.: G01N 33/574, G01N 33/74

(54) **METHOD FOR PREDICTING THE RISK OF GETTING BREAST CANCER IN A FEMALE SUBJECT**
VERFAHREN ZUR VORHERSAGE DES BRUSTKREBSRISIKOS BEI EINEM WEIBLICHEN PERSON
PROCÉDÉ DE PRÉDICTION DU RISQUE DE CANCER DU SEIN CHEZ UNE FEMME

(30) Priority: 08.03.2012 EP 12158679; 08.03.2012 US 201261608346 P; 20.04.2012 EP 12165054
(43) Date of publication of application: 14.01.2015
(73) Proprietor: SphingoTec GmbH, 16761 Hennigsdorf (DE)
(72) Inventor: BERGMANN, Andreas, 13465 Berlin (DE); MELANDER, Olle, S-20502 Malmö (SE)
(74) Representative: Kilger, Ute
(86) International application number: PCT/EP2013/054800
(87) International publication number: WO 2013/132089

(56) References cited:
- WO-A1-2009/044561
- WO-A1-2010/079158
- WO-A2-2007/093373
- US-A1- 2006 062 729
- US-A1- 2008 280 306
- CARRAWAY R.E. ET AL.: "Involvement of neurotensin in cancer growth: Evidence, mechanisms and development of diagnostic tools", PEPTIDES, vol. 27, no. 10, October 2006 (2006-10), pages 2445-2460, XP027957619, cited in the application
- ERNST A. ET AL.: "Proneurotensin 1-117, a stable neurotensin precursor fragment identified in human circulation", PEPTIDES, vol. 27, no. 7, July 2006 (2006-07), pages 1787-1793, XP027957440, cited in the application
- KITABGI P. ET AL.: "Differential processing of pro-neurotensin/neuromedin N and relationship to pro-hormone convertases", PEPTIDES, vol. 27, no. 10, October 2006 (2006-10), pages 2508-2514, XP027957626,
- MUSTAIN W.C. ET AL.: "The role of neurotensin in physiologic and pathologic processes", CURR. OPIN. ENDOCRINOL. DIABETES OBES., vol. 18, no. 1, February 2011 (2011-02), pages 75-82, XP009165711,
- MYERS R.M. ET AL.: "Cancer, chemistry, and the cell: Molecules that interact with the neurotensin receptors", ACS CHEM. BIOL., vol. 4, no. 7, 17 July 2009 (2009-07-17), pages 503-525, XP55089713,
- WANG J.-G. ET AL.: "Pancreatic cancer bears overexpression of neurotensin and neurotensin receptor subtype-1 and SR 48692 counteracts neurotensin induced cell proliferation in human pancreatic ductal carcinoma cell line PANC-1", NEUROPEPTIDES, vol. 45, no. 2, April 2011 (2011-04), pages 151-156, XP028155451,
- ROVÈRE C. ET AL.: "Pro-neurotensin/neuromedin N expression and processing in human colon cancer cell lines", BIOCHEM. BIOPHYS. RES. COMM., vol. 246, no. 1, May 1998 (1998-05), pages 155-159, XP55089053,
- MELANDER O. ET AL.: "Plasma proneurotensin and incidence of diabetes, cardiovascular disease, breast cancer, and mortality", JAMA, vol. 308, no. 14, 10 October 2012 (2012-10-10), pages 1469-1475, XP009165659,

## Description

Subject matter of the present invention is a method for predicting the risk of getting cancer in a female subject that does not suffer from cancer comprising:
- determining the level of pro-neurotensin 1-117 or fragments thereof of at least 5 amino acids or pro-neurotensin 1-117 comprising peptides by an immuno-assay or determining the level of pro-neurotensin 1-117 in a bodily fluid obtained from said female subject; and
- correlating said level of pro-neurotensin 1-117 or fragments thereof or pro-neurotensin 1-117 comprising peptides with the risk for getting cancer, wherein an elevated level is predictive for an enhanced risk of getting cancer, and
wherein said cancer is breast cancer and,
wherein said pro-neurotensin 1-117 is a peptide according to SEQ ID NO: 5, and
wherein said pro-neurotensin 1-117 comprising peptides are selected from a group of peptides comprising the peptide sequences of SEQ ID NO: 1, 2, 6 and 7,
and, wherein the body fluid is taken from a fasting female subject.

Neurotensin is a 13-amino acid neuropeptide derived from the prepro-neurotensin precursor and stochiometrically released together with the stable 117-amino acid peptide pro-neurotensin (P-NT) and the mature hormone binds to three different receptors, neurotensin receptor 1 and 2 (Ntsr1 and Ntsr2), which are G-protein coupled receptors and neurotensin receptor 3 (Ntsr3) which is non-G-protein coupled and also known as Sortillin-1 (SORT1).

Neurotensin is released peripherally from the small intestine as well as centrally from the hypothalamus. The peripheral secretion of neurotensin is stimulated by food-intake, especially by fat, and is known to regulate gastrointestinal motility and pancreatic and biliary secretion. Interestingly, neurotensin is implicated in appetite control as an anorectic hormone as it acutely reduces food intake following both central (intracerebroventricular) and peripheral (intraperitoneal) injection in rats, an effect which seems mainly mediated through the neurotensin-1 receptor (Ntsr1). In obese as compared to normal-weight human subjects, postprandial plasma neurotensin concentration was reduced following a liquid fatty meal (Widen et al 1992, Reg peptides; Plasma concentrations of regulatory peptides in obesity following modified sham feeding (MSF) and a liquid test meal), suggesting regulation of neurotensin secretion is disturbed in obesity. However, no large study has investigated if and how neurotensin is related to measures of obesity. Interestingly, P-NT significantly increases after gastric by-pass (Roux-en-Y), an operation shown to lead to normoglycemia in the majority of obese type 2 diabetes patients, but it is not known whether neurotensin is implicated in the development diabetes mellitus in general. Furthermore, the neurotensin system has been implicated in development of coronary artery disease and myocardial infarction as variation of the Ntsr3 (SORT1) gene is one of the strongest common coronary artery diseases susceptibility genes known in humans.

The mechanistic link between obesity and cancer is largely unknown, however, one of the dominating theories is that excess of fat deposits leads to increased peripheral aromatization of androgens and thus elevated circulating estrogen levels. In addition, one of the hallmarks of obesity, hyperinsulinemia, has been shown to inhibit hepatic production of sexual hormone binding globulin (SHBG), thus increasing bioavailable levels of both estrogens and androgens suggesting ways through which obesity may increase the risk of common forms of sex-hormone driven forms of cancer such as breast and prostate cancer. Interestingly, both neurotensin and Ntsr1 expression is common in malignant ductal breast cancer tumors and experimentally pharmacological blockade or RNA silencing of the NTSR1 reduces tumour growth in mice.

The level of expression of neurotensin receptor 1 (NTSR1) in breast cancer cells has been used for determining the prognosis of a subject suffering from breast cancer (US 2011/0305633). Further, it is stated in by the same authors that no clear correlation has been described today between circulating neurotensin and the stages of pancreas, prostate or medullar thyroid tumors probably due to rapid clearance by the liver. Interestingly, it was found that in a series of 51 patients with invasive ductal breast cancer 91 % of all tumors were positive for neurotensin receptor 1 (NTSR1) but only 31 % of all tumors were positive for neurotensin in said tissue (Souaze et al., Cancer Research (2006), 66:(12) pages 6243-6249).

There is some evidence that neurotensin and neurotensin receptors participate in cancer growth, in particular in lung cancer, pancreatic cancer and colon cancer (Carraway et al., Peptides (2006), 27 2445-2460). It has been reported that levels of NT in sera of patients with pancreatic cancer were significantly enhanced (Picheon et al., Anticancer Research (1999), 19 1445-50). Interestingly this group found that NT levels fell with progression of the disease for both prostate an pancreatic cancer. In contrast thereto, Meggiato et al. (Tumori (1996), 82; 592-5,) found that plasma levels of NT were normal in pancreatic cancer but elevated in case where pancreatitis was diagnosed.

The use of vasoactive peptides for prediction of cancer risks in males has been reported by Belting *et al.*, Cancer, Epidemiology, Biomarkes & Prevention. MR-pro-ANP, MR-pro-ADM and copeptin was measured in the fasting plasma from participants of the Malmö Diet and Cancer Study that were free from cancer prior to the baseline exam in 1991 to 1994 (1768 males and 2293 females). The authors stated that among females, there was no relationship between biomarkers and cancer incidence.

WO 2010/079158 A1 discloses methods for the treatment, the prognostic assessment and the detection of breast cancer.

Carraway, R.E., and Plona, A.M. (Peptides 27 (2006) 2445 to 2460) discloses the involvement of neurotensin in cancer growth: Evidence mechanisms and development of diagnostic tools.

Ernst, A., et al.: Peptides, Vol. 27, No. 7, July 2006, pages 1787 to 1793 disclose that pro-neurotensin 1 to 117 is a stable neurotensin precursor fragment identified in human circulation.

A subject of the present invention was to investigate the prognostic power of NT for the prediction of cancer incidence and the prediction of the risk of reoccurrence of cancer. To address this issue, stable fragments of pro-neurotensin in fasting plasma were measured in said Swedish prospective cohort study (Malmö Diet and Cancer Study) and related baseline level of this biomarker to breast-cancer incidence during 15 years of follow-up.

Surprisingly, it has been shown that neurotensin is a powerful and highly significant biomarker for woman for predicting the risk of getting cancer in a female subject that does not suffer from cancer.

Thus, subject matter of the present invention is a method for predicting the risk of getting cancer in a female subject that does not suffer from cancer:
comprising:
- determining the level of pro-neurotensin 1-117 or fragments thereof of at least 5 amino acids or pro-neurotensin 1-117 comprising peptides by an immuno-assay or determining the level of pro-neurotensin 1-117 in a bodily fluid obtained from said female subject; and
- correlating said level of pro-neurotensin 1-117 or fragments thereof or pro-neurotensin 1-117 comprising peptides with the risk for getting cancer, wherein an elevated level is predictive for an enhanced risk of getting cancer, and
wherein said cancer is breast cancer and,
wherein said pro-neurotensin 1-117 is a peptide according to SEQ ID NO: 5, and
wherein said pro-neurotensin 1-117 comprising peptides are selected from a group of peptides comprising the peptide sequences of SEQ ID NO: 1, 2, 6 and 7,
and, wherein the body fluid is taken from a fasting female subject.

The level of pro-neurotensin 1-117 or fragments thereof of at least 5 amino acids or pro-neurotensin 1-117 comprising peptides in a bodily fluid obtained from said female subject that is predictive for the risk of getting cancer in said female subject that does not suffer from cancer is released from the small intestine. It may not be released from cancer cells as the female subject does not suffer from cancer. The release of neurotensin from the small intestine is stimulated by food intake, especially by fat, and is known to regulate gastrointestinal motility and pancreatic and biliary secretion. Pro-neurotensin 1 -117 and fragments thereof or pro-neurotensin 1-117 comprising peptides are used as a surrogate marker for the released neurotensin as neurotensin and pro-neurotensin 1 -117 and fragments thereof or pro-neurotensin 1-117 comprising peptides are released in equimolar amounts from pro-neurotensin.

It is the surprising finding of the present invention that the peripheral secretion of neurotensin/pro-neurotensin 1-117 or fragments thereof of at least 5 amino acids or pro-neurotensin 1-117 comprising peptides as defined in the claims is indicative for the susceptibility of a female subject to acquire cancer. Thus, dietary measures as reduction of that uptake may lower said risk in said female subject. It is known from literature that neurotensin and neurotensin receptor is overexpressed in tissue of cancer that is selected from the group comprising breast cancer, lung cancer, pancreatic cancer and colon cancer. Thus, elevated levels of neurotensin/pro-neurotensin 1-117 or fragments thereof of at least 5 amino acids or pro-neurotensin 1-117 comprising peptides in bodily fluids as defined in the claims enhance the risk of getting a cancer where neurotensin and/ or pro-neurotensin plays a role, i.e. in cancers where neurotensin receptor is overexpressed in said cancer cells, e.g. in breast cancer.

Thus, subject matter of the present invention is the determination of susceptibility of a woman to acquire breast cancer.

Data obtained in the present study revealed also a correlation between the risk of getting cancer in male subjects with the level of pro-neurotensin or fragments thereof of at least 5 amino acids in a bodily fluid obtained from said male subject; this correlation however, was not that significant for the present data set. Thus, there is a value for the method disclosed herein also for male subjects but in the present study the observed effect was not as strong for males as compared to females.

The term "subject" as used herein refers to a living female organism. Preferably, herein the subject is a female human subject.

The correlation between the level of pro-neurotensin 1-117 or fragments thereof of at least 5 amino acids or pro-neurotensin 1-117 comprising peptides as defined in the claims in a bodily fluid obtained from said female subject and the risk of getting cancer is continuous, i.e. the higher the level the higher the risk. This can be seen from the data e.g. in Table 6. In comparison to the first quartile the second, third and fourth quartile exhibits higher Hazard Risks respectively.

For the sake of practicability the person skilled in the art may use threshold(s).

Thus, the term "elevated level" may mean a level above a threshold level.

According to the invention the level of pro-neurotensin or fragments thereof of at least 5 amino acids or pro-neurotensin 1-117 comprising peptides in a bodily fluid is the fasting level of pro-neurotensin or fragments thereof of at least 5 amino acid or pro-neurotensin 1-117 comprising peptides as defined in the claims. Fasting level means no food uptake 12 h prior blood sampling.

A bodily fluid may be selected from the group comprising blood, serum, plasma, urine, cerebrospinal liquid (csf), and saliva.

In one embodiment of the invention said female subject has never had a diagnosed cancer at the time the sample of bodily fluid is taken from said female subject.

In another embodiment said female subject has been diagnosed before with having cancer and has been cured at the time the sample of bodily fluid is taken from said female subject and the risk of reoccurrence of getting cancer is determined.

In one embodiment of the invention said female subject has been diagnosed as having at a cardiovascular disease or diabetes at the time the sample of bodily fluid is taken from said female subject.

Said cardiovascular disease may be selected from the group comprising heart failure, atherosclerosis and hypertension.

In another embodiment of the invention said female subject has been diagnosed as having at diabetes type 2 at the time the sample of bodily fluid is taken from said female subject.

The definition of diabetes is as follows: a history of physical diagnosis or being on anti-diabetic medication or having a fasting whole blood glucose >/= 6.1 mmol/l (note this is = 7.0 mmol/l in plasma) at the baseline examination.

The definition of normotensive/ high blood pressure (HBP) is as follows:
HBP: Systolic BP>/=140 mmHg or Diastolic BP >/=90 mmHg or being on antihypertensive medications. Subjects having normal blood pressure (BP) are all other subjects, i.e subjects with Systolic BP<140 and Diastolic BP <90 and not being on antihypertensive medications.

The present data suggest a strong correlation between the level of pro-neurotensin or fragments thereof as defined in the claims with breast cancer in women with no prevalent diabetes, no prevalent breast cancer and no prevalent cardiovascular disease.

The present data also suggest a strong correlation between the level of pro-neurotensin or fragments thereof as defined in the claims with breast cancer in hypertensive women, which is a common high-risk group for cardiovascular disease.

Furthermore, the present data also suggest a strong correlation between the level of pro-neurotensin or fragments thereof as defined in the claims with breast cancer in normotensive women. Further, the present data suggest a strong correlation between the level of pro-neurotensin or fragments thereof as defined in the claims with breast cancer in diabetic women.

In a specific embodiment of the method according to the invention additionally at least one clinical parameter is determined selected from the group comprising: age, presence of diabetes mellitus, current smoking.

Fragments of pro-neurotensin that may be determined in a bodily fluid may be e.g. selected from the group of the following fragments:

SEQ ID NO: 1 (pro-neurotensin 1-147)

SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY ENKPRRPYIL KRDSYYY

SEQ ID NO: 2 (pro-neurotensin 1-125 (large neuromedin N))

SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI KR KIPYIL

SEQ ID NO: 3 (neuromedin N:), which is disclosed, but not claimed
   KIPYIL
SEQ ID NO: 4 (neurotensin), which is disclosed, but not claimed
   pyroQLYENKPRRP YIL

SEQ ID NO: 5 (pro-neurotensin 1-117)

SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVI

SEQ ID NO: 6 (pro-neurotensin 1-132)

SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYILKRQLY EN

Seq ID No 7: (pro-neurotensin 1-125)

SDSEEEMKAL EADFLTNMHT SKISKAHVPS WKMTLLNVCS LVNNLNSPAE
ETGEVHEEEL VARRKLPTAL DGFSLEAMLT IYQLHKICHS RAFQHWELIQ
EDILDTGNDK NGKEEVIKRK IPYIL

SEQ ID NO: 8 (pro-neurotensin 120-140), which is disclosed, but not claimed
   KIPYILKRQL YENKPRRPYI L
SEQ ID NO: 9 (pro-neurotensin 120-147), which is disclosed, but not claimed
   KIPYILKRQL YENKPRRPYIL KRDSYYY
SEQ ID NO: 10 (pro-neurotensin 128-147), which is disclosed, but not claimed
   QLYENKPRRP YILKRDSYYY

In a more specific embodiment of the method according to the present invention the level of pro-neurotensin 1-117 is determined.

In accordance with the invention, the level of pro-neurotensin is measured with an immunoassay. More specifically an immunoassay is used as described in Ernst et al. (Peptides (2006), (27) 1787-1793). An immunoassay that may be useful for determining the level of pro-neurotensin or fragments thereof of at least 5 amino acids may comprise the steps as outlined in Example 2. All thresholds and values have to be seen in correlation to the test and the calibration used according to Example 2. A person skilled in the art may know that the absolute value of a threshold might be influenced by the calibration used. This means that all values and thresholds given herein are to be understood in context of the calibration used in herein (Example 2). A human P-NT-calibrator is available by ICI-Diagnostics, Berlin, Germany. Alternatively, the assay may also be calibrated by synthetic or recombinant P-NT 1-117 or fragments thereof (see also Ernst et. al, 2006).

The threshold for determining the risk of getting breast cancer in a female subject according to the methods of the present invention is above 78 pmol/l PNT, preferred 100 pmol/l, more preferred 150 pmol/l. In a specific embodiment said threshold is about 100 pmol/l. These thresholds are related to the above mentioned calibration method. A P-NT value above said threshold means that the subject has an enhanced risk of getting cancer or has already cancer.

In one embodiment of the invention said method is performed more than once in order to monitor the risk of getting breast cancer in a female subject or in order to monitor the course of treatment. In one specific embodiment said monitoring is performed in order to evaluate the response of said female subject to preventive and/or therapeutic measures taken.

In one embodiment of the invention the method is used in order to stratify said female subjects into risk groups.

Disclosed is also a method for predicting the risk of getting cancer in a female or identifying a female subject having an enhanced risk for getting cancer according to any of the preceding embodiments, wherein the level of pro-neurotensin or fragments thereof of at least 5 amino acids in a bodily fluid obtained from said female subject either alone or in conjunction with other prognostically useful laboratory or clinical parameters is used for the prediction of a subject's risk for getting an adverse event by a method which may be selected from the following alternatives:
- Comparison with the median of the level of pro-neurotensin or fragments thereof of at least 5 amino acids in a bodily fluid obtained from said female subject in an ensemble of pre-determined samples in a population of "healthy" or "apparently healthy" subjects,
- Comparison with a quantile of the level of pro-neurotensin or fragments thereof of at least 5 amino acids in a bodily fluid obtained from said female subject in an ensemble of pre-determined samples in a population of "healthy" or "apparently healthy" subjects,
- Calculation based on Cox Proportional Hazards analysis or by using Risk index calculations such as the NRI (Net Reclassification Index) or the IDI (Integrated Discrimination Index).

According to the method of the present invention said female subject's risk for developing breast cancer may be reclassified as a consequence of the determination of the level of pro-neurotensin 1-117 or fragments thereof of at least 5 amino acids or pro-neurotensin 1-117 as defined in the claims comprising peptides in a bodily fluid obtained from said female subject.

Disclosed is also point-of-care device for performing a method according to the invention.

Disclosed is also a binder to neurotensin or to a neurotensin receptor, for the use in prevention or therapy of cancer in a female subject.

The herein disclosed binder reduces the bioactivity of neurotensin to 70 % or less.

According to the invention the cancer is breast cancer.

Herein disclosed is a binder to neurotensin that is selected from the group consisting of antibodies e.g. IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains,*e.g.* Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines.

Herein disclosed is a binder to a neurotensin receptor that is selected from the group consisting of antibodies e.g. IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains,*e.g.* Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, e.g. from a different class than G; single-domain antibodies, *e.g.* nanobodies derived from camelid or fish immunoglobulines, or a peptide antagonist e.g. [D-Trp¹¹]-Neurotensin, [Tyr(Me)¹¹]-Neurotensin (e.g. described by Quiron et al.), or a non-peptide antagonist, e.g. Levocabastine, SR-48692 (NTS1 selective), SR-142948 (unselective), SR-142948A, CP 96345, [3H]SR-48692, SR 48692, SR-48527 and SR-49711, or a binder scaffold e.g. tetranectin-based non-Ig scaffolds (e.g. described in US 2010/0028995), fibronectin scaffolds (e.g. described in EP 1266 025; lipocalin-based scaffolds ((e.g. described in WO 2011/154420); ubiquitin scaffolds (e.g. described in WO 2011/073214), transferring scaffolds (e.g. described in US 2004/0023334), protein A scaffolds (e.g. described in EP 2231860), ankyrin repeat based scaffolds (e.g. described in WO 2010/060748), microproteins preferably microproteins forming a cystine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867).

Another preventive measure may be the reduction of neurotensin secretion e.g. by watching a low fat diet or other dietary measurements that may reduce neurotensin secretion.

### Examples

### Example 1

### Development of Antibodies

### Peptides/ conjugates for Immunization:

Peptides for immunization were synthesized (JPT Technologies, Berlin, Germany) with an additional N-terminal Cystein residue for conjugation of the peptides to bovine serum albumin (BSA). The peptides were covalently linked to BSA by using SulfoLink-Coupling gel (Perbio-science, Bonn, Germany). The coupling procedure was performed according to the manual of Perbio.
Labelled antibody (LA) peptide (P-NT 1-19):
H-CSDSEEEMKALEADFLTNMH-NH2
Solid phase antibody (SPA) peptide (P-NT 44-62):
H-CNLNSPAEETGEVHEEELVA-NH2
The antibodies were generated according to the following method:
A BALB/c mouse were immunized with 100 µg peptide-BSA-conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitonal and one intra venous injection.

Splenocytes from the immunized mouse and cells of the myeloma cell line SP2/0 were fused with 1 ml 50 % polyethylene glycol for 30 s at 37 °C. After washing, the cells were seeded in 96-well cell culture plates. Hybrid clones were selected by growing in HAT medium [RPMI 1640 culture medium supplemented with 20 % fetal calf serum and HAT-supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined.

(Lane, R.D. "A short-duration polyethylene glycol fusiontechnique for increasing production of monoclonal antibody-secreting hybridomas", J. Immunol. Meth. 81: 223-228; (1985), Ziegler, B. et al. "Glutamate decarboxylase (GAD) is not detectable on the surface of rat islet cells examined by cytofluorometry and complement-dependent antibody-mediated cytotoxicity of monoclonal GAD antibodies", Horm. Metab. Res. 28: 11-15, (1996)).

### Monoclonal antibody production

Antibodies were produced via standard antibody production methods (Marx et al., Monoclonal Antibody Production (1997), ATLA 25, 121) and purified via Protein A-chromatography. The antibody purities were > 95 % based on SDS gel electrophoresis analysis.

### Example 2

### Immunoassay for the quantification of human pro-neurotensin

The technology used was a sandwich coated tube luminescence immunoassay, based on Acridinium ester labelling.

Labelled compound (tracer): 100 µg (100 µl) LA (1 mg/ml in PBS, pH 7.4, was mixed with 10 µl Acridinium NHS-ester (1 mg/ml in acetonitrile, InVent GmbH, Germany) (EP 0353971) and incubated for 20 min at room temperature. Labelled LA was purified by gel-filtration HPLC on Bio-Sil SEC 400-5 (Bio-Rad Laboratories, Inc., USA) The purified LA was diluted in (300 mmol/l potassiumphosphate, 100 mmol/l NaCl, 10 mmol/l Na-EDTA, 5 g/l bovine serum albumin, pH 7.0). The final concentration was approx. 800.000 relative light units (RLU) of labelled compound (approx. 20 ng labeled antibody) per 200 µl. Acridiniumester chemiluminescence was measured by using an AutoLumat LB 953 (Berthold Technologies GmbH & Co. KG).

Solid phase: Polystyrene tubes (Greiner Bio-One International AG, Austria) were coated (18 h at room temperature) with SPA (1.5 µg SPA/0.3 ml 100 mmol/l NaCl, 50 mmol/l Tris/HCl, pH 7.8). After blocking with 5 % bovine serum albumine, the tubes were washed with PBS, pH 7.4 and vakuum dried.Calibration:
The assay was calibrated, using dilutions of P-NT-containing human serum. A pool of human sera with high P-NT-immunoreactivity (InVent Diagostika, Hennigsdorf, Germany) was diluted with horse serum (Biochrom AG, Deutschland) (assay standards).

The standards were calibrated by use of the human ProNT-calibrator (ICI-Diagnostics, Berlin, Germany). Alternatively, the assay may be calibrated by synthetic or recombinant P-NT 1-117 or fragments thereof (see also Ernst *et al.*, 2006).

### ProNT Immunoassay:

50 µl of sample (or calibrator) was pipetted into SPA coated tubes, after adding labeleld LA (200ul), the tubes were incubated for 16-22 h at 18-25 °C. Unbound tracer was removed by washing 5 times (each 1 ml) with washing solution (20 mmol/l PBS, pH 7.4, 0.1 % Triton X-100).

Tube-bound LA was measured by using the LB 953.

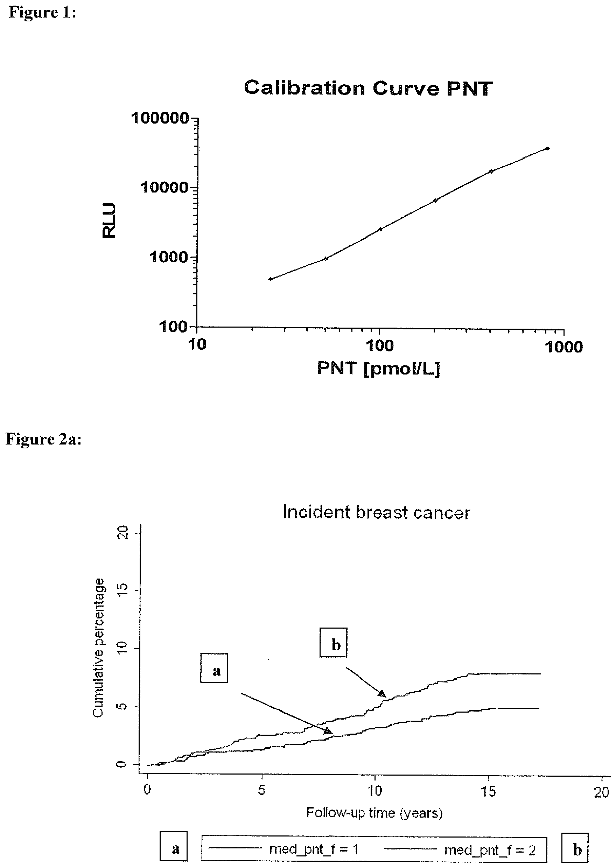

Figure 1 shows a typical P-NT dose/ signal curve.

### Example 3

### Population study

### Methods

We measured P-NT in fasting plasma from 2559 female participants of the population based Malmö Diet and Cancer Study baseline exam in 1991-1994 (men age 58 ± 6 years and 59 % females). We used multivariable adjusted (all traditional cardiovascular risk factors, diabetes risk factors and in analyses of cancer also heredity for cancer) Cox proportional hazards models to relate baseline P-NT (hazard ratio per each standard deviation increase of log-transformed P-NT) to the time to the first event of each of the studied endpoints during a median follow-up time of more than 12 years. Endpoints were retrieved through the Swedish National Hospital Discharge Registry, the Swedish Myocardial Infarction Registry, the Stroke in Malmö Registry and the Swedish Cancer Registry. Retrieval of endpoints through these registries has been validated and found to be accurate (see also Belting et al Cancer Epidemiol Biomarkers Prev; 1-10. _2012 AACR).Clinical characteristics of females in the study

| Descriptive Statistics | | | |
|---|---|---|---|
| **Table 1** | | | |
| | N | Mean | Std. Deviation |
| Age at MDCS screening | 2559 | 57.554 | 5.9403 |
| Systolic blood pressure (mmHg) | 2559 | 140.50 | 19.311 |
| Diastolic blood pressure (mmHg) | 2559 | 85.65 | 9.117 |
| body-mass-index (weight/kgxkg) | 2559 | 25.5196 | 4.19083 |
| WAIST (cm) | 2559 | 76.99 | 10.245 |
| Glucose (mmol/l) | 2559 | 5.0418 | 1.21798 |
| Triglycerides (mmol/l) | 2559 | 1.2245 | .58404 |
| High density lipoprotein (mmol/l) | 2559 | 1.5123 | .36949 |
| Low density lipoprotein (mmol/l) | 2559 | 4.2016 | 1.04762 |
| P-INSULIN | 2512 | 7.223 | 5.4223 |
| P-NT (pmol/l) | 2559 | 125.60633 | 77.681673 |

**Table 2**

| Q+Diary: Anti hypertension treatment (C02,C03,C07,C08,C09) at baseline according to questionnaire or diary book | | | | | |
|---|---|---|---|---|---|
| | | Frequency | Percent | Valid Percent | Cumulative Percent |
| Valid | No | 2173 | 84.9 | 84.9 | 84.9 |
| | Yes | 386 | 15.1 | 15.1 | 100.0 |
| | | | | | |
| | Total | 2559 | 100.0 | 100.0 | |

**Table 3**

| DIAB MELL (fb >6.0 mmol/l or pos Q DM) | | | | | |
|---|---|---|---|---|---|
| | | Frequency | Percent | Valid Percent | Cumulative Percent |
| Valid | no | 2396 | 93.6 | 93.6 | 93.6 |
| | yes | 163 | 6.4 | 6.4 | 100.0 |
| | Total | 2559 | 100.0 | 100.0 | |

**Table 4**

| current_smoker0 | | | | | |
|---|---|---|---|---|---|
| | | Frequency | Percent | Valid Percent | Cumulative Percent |
| Valid | .00 | 1906 | 74.5 | 74.5 | 74.5 |
| | 1.00 | 653 | 25.5 | 25.5 | 100.0 |
| | Total | 25 5 9 | 100.0 | 100.0 | |

**Table 5**

| QUARTILES OF PNT IN WOMEN: | | | | |
|---|---|---|---|---|
| PNT (pmol/l) | | | | |
| Percentile group of PNT (pmol/l) | N | Median | Minimum | Maximum |
| 1 | 639 | 62.37000 | 5.100 | 78.580 |
| 2 | 639 | 92.07000 | 78.610 | 108.770 |
| 3 | 641 | 125.07000 | 108.960 | 150.000 |
| 4 | 640 | 194.38500 | 150.050 | 1154.520 |
| Total | 2559 | 108.96000 | 5.100 | 1154.520 |

Quartile-concentrations were almost identical in all shown subgroup analysis.

### P-NT and prediction of breast cancer

We assessed the relationship between P-NT and breast cancer (Table 6). There was a strong relationship between P-NT and breast cancer in females. In a fully adjusted model each SD increase of P-NT was associated with a 35 % increased risk of future breast cancer and the top versus bottom quartile of P-NT identified a more than 2-fold difference in risk of breast cancer (see table 6 and table 7).

**Table 6**

| BREAST CANCER | | | | | | | |
|---|---|---|---|---|---|---|---|
| | HR per 1 SD | P-value | Quartile 1 | Quartile 2 | Quartile 3 | Quartile 4 | P for trend |
| Women (2140 / 135) | 1.36 (1.14-1.59) | <0.001 | 1.0 (ref) | 1.20 (0.69-2.07) | 1.55 (0.93-2.61) | 2.08 (1.27-3.42) | 0.002 |

| | | | | | | | |
|---|---|---|---|---|---|---|---|
| Multivariate Cox proportional Hazards models for baseline P-NT versus incidence of breast cancer | | | | | | | |

**Table 7**

| BREAST CANCER-FEMALES | | | | | | | | |
|---|---|---|---|---|---|---|---|---|
| (females with any cancer history were excluded, 2140 women/ 135 events) | | | | | | | | |
| Variables in the equation | | | | | | | 95.0% CI for Exp(B) | |
| | B | SE | Wald | df | Sig. | Exp(B) | Lower | Upper |
| AGE | .004 | .017 | .058 | 1 | .810 | 1.004 | .971 | 1.039 |
| AHT_B | -.014 | .262 | .003 | 1 | .958 | .986 | .590 | 1.648 |
| SBP_B | -.001 | .005 | .013 | 1 | .911 | .999 | .989 | 1.010 |
| BMI_B | .046 | .025 | 3.230 | 1 | .072 | 1.047 | .996 | 1.100 |
| HDL_B | -.065 | .263 | .061 | 1 | .805 | .937 | .560 | 1.568 |
| LDL_B | -.080 | .093 | .752 | 1 | .386 | .923 | .769 | 1.107 |
| current_smoker0 | .298 | .201 | 2.202 | 1 | .138 | 1.347 | .909 | 1.996 |
| LNIN S | -.368 | .204 | 3.234 | 1 | .072 | .692 | .464 | 1.034 |
| DM_B | -.103 | .413 | .062 | 1 | .803 | .902 | .401 | 2.028 |
| | | | | | | | | |
| HER_CANCER_0 | .014 | .179 | .006 | 1 | .936 | 1.015 | .714 | 1.441 |
| **ZLN_PNT** | **.307** | **.087** | **12.520** | **1** | **.000** | **1.360** | **1.147** | **1.612** |

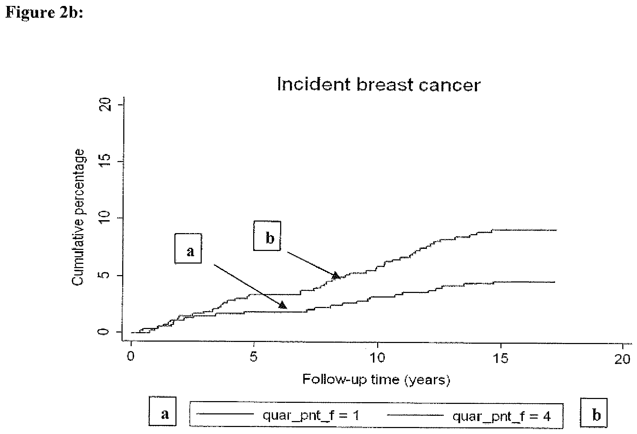

Figure.2: Kaplan Meier graphs, illustrating the cumulative breast cancer diagnosis in women a) below and above P-NT median (109 pmol/l) and b) quartile (Q) 1 vs quartile 4 (below 79 pmol/l and above 150 pmol/l)
Legend figure 2a and 2b: Increased P-NT indicates a long term increased risk of breast cancer development. Since any women with cancer history at day of baseline (blood sampling) were excluded, PNT is highly predictive for future breast cancer development. Over all, women from Q 4 have an 2 times higher risk to develop breast cancer than women from Q 1.

### Subgroup analysis in women with and without diabetes/ high blood pressure at baseline.

Definition Diabetes: A history of physical diagnosis or being on anti-diabetic medication or having a fasting whole blood glucose >/= 6.1 mmol/l (note this is = 7.0 mmol/l in plasma) at the baseline examination.

### Definition normotensive/high blood pressure (HBP):

HBP: SystolicBP>/=140 mmHg or Diastolic BP >/=90 mmHg or being on antihypertensive medications. Normal BP: all other subjects.

Using the same variables in the equation, we investigated different subgroups of women for prediction of cancer (women with any cancer history were excluded): Table 8

**Table 8**

| Subgroup | No of subjects | No of events | Hazard risk per 1SD PNT | Significance (p-value) |
|---|---|---|---|---|
| all | 2140 | 135 | 36% | <0.001 |
| IIBP Women | 1294 | 79 | 26,3% | <0,05 |
| Normotensive women | 846 | 56 | 52% | 0,001 |
| Women w/o history of CV-events and w/o Diabetes | 1984 | 128 | 35% | <0,001 |
| Normotensive women w/o history of CV-events and w/o Diabetes | 891 | 52 | 45% | 0.006 |

In all subgroups P-NT is significantly predictive for breast cancer development. The predictive power of PNT was slightly stronger in healthier women.

### Example 4

### Reclassification of woman into risk groups

### Methods:

We calculated model c-statistics and reclassification across 10-year predicted risk categories for the different events (<5%, >=5-10%, >=10-20% and >=20%, respectively) with Net Reclassification Improvement (NRI) for models with and without P-NT.

Pencina MJ, D'Agostino RB. Overall C as a measure of discrimination in survival analysis: model specific population value and confidence interval estimation. Stat Med. Jul 15 2004;23(13):2109-2123.

Pencina MJ, D'Agostino RB, Sr., D'Agostino RB, Jr., Vasan RS. Evaluating the added predictive ability of a new marker: from area under the ROC curve to reclassification and beyond. Stat Med. Jan 30 2008;27(2):157-172; discussion 207-112.

Ridker PM, Buring JE, Rifai N, Cook NR. Development and validation of improved algorithms for the assessment of global cardiovascular risk in women: the Reynolds Risk Score. Jama. Feb 14 2007;297(6):611-619.

All analyses were performed with Stata software version 11 (StataCorp, College Station, Texas).

A two-sided P-value of <.05 was considered statistically significant.

### Results:

### Discrimination and risk reclassification in females

Adding P-NT on top of all breast cancer risk factors, significantly correctly reclassified women across categories of risk of 10 year breast cancer outcome with an NRI of 25%. Of women who later developed breast cancer, 34% were moved to a higher category of risk whereas 27% of women who did not develop breast cancer were moved to a lower category of risk. The C-statistic for breast cancer increased after adding P-NT on top of known breast cancer risk factors by 4.7% **(Table 9)**.

**Table 9:**

| Breast cancer (n=1657, 113 events) | | |
|---|---|---|
| | C-statistic | NRI |
| Basic model | 61.4% | ref |
| +pro-neurotensin | 66.1% | 25% (P<0.001) |

| | | |
|---|---|---|
| *Clinical NRI refers to correct movements up and down from the intermediate category of risk (10-20% 10-year risk) after addition of pro-neurotensin to the basic models. | | |

### Example 5

Association of PNT and prediction of breast cancer in comparison to other cancers in female and in comparison to prediction of cancer in males
There is a highly significant association of PNT and prediction of breast cancers. There is no significant association in cervix cancer, uterus cancer and ovary cancer.

This supports the present finding that elevated levels of neurotensin/proneurotensin 1-117 in bodily fluids enhance the risk of getting cancer where neurotensin and/or proneurotensin plays a role, i.e. in cancers where neurotensin receptor is over expressed in said cancer cells.

Further, there is no significant association of PNT and prediction of prostate cancer.

**Table 10: Association of PNT and prediction of cancer:**

| female | | | | |
|---|---|---|---|---|
| No subjects at baseline: 2240 | No of events | p-value for difference at baseline | significance | |
| Breast cancer | 128 | 0,001 | +++ | |
| Cervix cancer | 18 | 0,997 | --- | |
| Uterus cancer | 18 | 0,366 | --- | |
| Ovary cancer | 15 | 0,94 | --- | |
| | | | | |

| male | | | | |
|---|---|---|---|---|
| No of subjects at baseline: 1911 | | | | |
| Prostate cancer | 163 | 0,310 | --- | |

### Figure description

### Figure 1 shows a typical P-NT dose/ signal curve

Legend figure 2a and 2b: Increased P-NT indicates a long term increased risk of breast cancer development. Since any women with cancer history at day of baseline (blood sampling) were excluded, PNT is highly predictive for future breast cancer development. Over all, women from Q 4 have an 2 times higher risk to develop breast cancer than women from Q 1

## Claims

1. An *in vitro* method for predicting the risk of getting cancer in a female subject that does not suffer from cancer comprising:
• determining the level of pro-neurotensin 1-117 or fragments thereof of at least 5 amino acids or pro-neurotensin 1-117 comprising peptides by an immuno-assay or determining the level of pro-neurotensin 1-117 in a bodily fluid obtained from said female subject; and
• correlating said level of pro-neurotensin 1-117 or fragments thereof or pro-neurotensin 1-117 comprising peptides with the risk of getting cancer, wherein an elevated level is predictive for an enhanced risk of getting cancer, and
wherein said cancer is breast cancer and,
wherein said pro-neurotensin 1-117 is a peptide according to SEQ ID NO 5, and
wherein said pro-neurotensin 1-117 comprising peptides are selected from a group of peptides comprising the peptide sequences of SEQ ID NO 1, 2, 6 and 7, and
wherein the bodily fluid is taken from a fasting female subject.

2. A method according to claim 1, wherein elevated level means a level above a threshold level.

3. A method according to any of claims 1 or 2, wherein said female subject has never had a history of diagnosis of cancer at the time the sample of bodily fluid is taken from said female subject.

4. A method according to claims 1 to 3, wherein said female subject has had a history of diagnosis of cancer and has been cured at the time the sample of bodily fluid is taken from said female subject and the risk of reoccurrence of getting breast cancer is determined.

5. A method according to claims 1 to 4, wherein at the time the sample of bodily fluid is taken from said female subject, said female subject has been diagnosed as having a cardiovascular disease or diabetes.

6. A method according to claim 5, wherein at the time the sample of bodily fluid is taken from said female subject, said cardiovascular disease may be selected from the group comprising heart failure, atherosclerosis, and hypertension.

7. A method according to claims 5, wherein at the time the sample of bodily fluid is taken from said female subject, said female subject has been diagnosed as having diabetes type 2.

8. A method according to claims 1 to 7, wherein additionally at least one clinical parameter is determined selected from the group comprising: age, presence of diabetes mellitus, current smoking.

9. A method according to any of the preceding claims, wherein the level of pro-neurotensin 1-117 is determined.

10. A method according to any of the preceding claims, wherein the level of pro-neurotensin 1-117 or fragments thereof or pro-neurotensin 1-117 comprising peptides is measured with an immunoassay.

11. A method according to any of claims 1 - 10, wherein said method is performed more than once in order to monitor the risk of getting breast cancer in a female subject.

12. A method according to claim 11, wherein said monitoring is performed in order to evaluate the response of said female subject to preventive and/or therapeutic measures taken.

13. A method according to any of claims 1 to 12 in order to stratify said female subjects into risk groups.

14. A method according to claim 13, wherein said female subject is reclassified as a consequence of the determination of the level of pro-neurotensin 1-117 or fragments thereof of at least 5 amino acids or pro-neurotensin 1-117 comprising peptides in a bodily fluid obtained from said female subject.

## Patentansprüche

1. *In-vitro*-Verfahren zur Vorhersage des Krebsrisikos bei einer weiblichen Person, die nicht an Krebs leidet, umfassend:
• Bestimmung des Wertes von Pro-Neurotensin 1-117 oder Fragmenten davon, die mindestens 5 Aminosäuren umfassen, oder von Pro-Neurotensin 1-117 umfassenden Peptiden mithilfe eines Immunassays oder Bestimmung des Wertes von Pro-Neurotensin 1-117 in einer der weiblichen Person entnommenen Körperflüssigkeit, und
• Korrelation dieses Wertes von Pro-Neurotensin 1-117 oder Fragmenten davon oder von Pro-Neurotensin 1-117 umfassenden Peptiden mit dem Krebsrisiko, wobei ein erhöhter Wert einem erhöhten Krebsrisiko entspricht, und
wobei der Krebs Brustkrebs ist, und
wobei das Pro-Neurotensin 1-117 ein Peptid nach SEQ ID NO: 5 ist, und
wobei die Pro-Neurotensin 1-117 umfassenden Peptide ausgewählt sind aus der Gruppe von Peptiden umfassend die Peptidsequenzen von SEQ ID NO 1, 2, 6 und 7, und
wobei die Körperflüssigkeit einer nüchternen weiblichen Person entnommen wird.

2. Verfahren nach Anspruch 1, wobei ein erhöhter Wert ein Wert über einem Schwellenwert bedeutet.

3. Verfahren nach einem der Ansprüche 1 oder 2, wobei bei der weiblichen Person zum Zeitpunkt der Entnahme der Körperflüssigkeit keine vorherigen Krebsdiagnosen vorliegen.

4. Verfahren nach den Ansprüchen 1 bis 3, wobei bei der weiblichen Person eine vorherige Krebsdiagnose vorlag, der Krebs zum Zeitpunkt der Entnahme der Körperflüssigkeit geheilt ist und das Risiko des Wiederauftretens von Brustkrebs bestimmt wird.

5. Verfahren nach den Ansprüchen 1 bis 4, wobei bei der weiblichen Person zum Zeitpunkt der Entnahme der Körperflüssigkeit eine kardiovaskuläre Erkrankung oder Diabetes diagnostiziert wurde.

6. Verfahren nach Anspruch 5, wobei bei der weiblichen Person zum Zeitpunkt der Entnahme der Körperflüssigkeit die kardiovaskuläre Erkrankung aus der Gruppe umfassend Herzversagen, Atherosklerose und Hypertonie ausgewählt werden kann.

7. Verfahren nach Anspruch 5, wobei bei der weiblichen Peson zum Zeitpunkt der Entnahme der Körperflüssigkeit Diabetes Typ 2 diagnostiziert wurde.

8. Verfahren nach den Ansprüchen 1 bis 7, wobei zusätzlich mindestens ein klinischer Parameter, ausgewählt aus der Gruppe umfassend Alter, Vorhandensein von Diabetes mellitus, derzeitigen Rauchgewohnheiten, bestimmt wird.

9. Verfahren nach einem der vorangehenden Ansprüche, wobei der Wert von Pro-Neurotensin 1-117 bestimmt wird.

10. Verfahren nach einem der vorangehenden Ansprüche, wobei der Wert von Pro-Neurotensin 1-117 oder Fragmenten davon oder von Pro-Neurotensin 1-117 umfassenden Peptiden mithilfe eines Immunoassays bestimmt wird.

11. Verfahren nach einem der Ansprüche 1 bis 10, wobei das Verfahren mehr als einmal durchgeführt wird, um das Brustkrebsrisiko bei einer weiblichen Person zu überwachen.

12. Verfahren nach Anspruch 11, wobei die Überwachung durchgeführt wird, um die Reaktion der weiblichen Person auf getroffene vorbeugende und/oder therapeutische Maßnahmen zu bewerten.

13. Verfahren nach einem der Ansprüche 1 bis 12, um die weiblichen Personen in Risikogruppen zu stratifizieren.

14. Verfahren nach Anspruch 13, wobei die weibliche Person infolge der Bestimmung des Wertes von Pro-Neurotensin 1-117 oder Fragmenten davon, die mindestens 5 Aminosäuren umfassen, oder von Pro-Neurotensin 1-117 umfassenden Peptiden in einer von der weiblichen Person entnommenen Körperflüssigkeit neu klassifiziert wird.

## Revendications

1. Méthode in vitro pour prédire le risque d'apparition d'un cancer chez un sujet de sexe féminin qui n'est pas atteint d'un cancer, ladite méthode comprenant :
• la détermination du taux de pro-neurotensine 1-117 ou de ses fragments d'au moins 5 acides aminés ou de pro-neurotensine 1-117 comprenant des peptides à l'aide d'un immuno-essai ou la détermination due taux de pro-neurotensine 1-117 dans un liquide corporel provenant dudit sujet de sexe féminin, et
• la corrélation dudit taux de pro-neurotensine 1-117 ou de ses fragments ou de pro-neurotensine 1-117 comprenant des peptides avec le risque de contracter un cancer, un taux élevé prédisant un risque accru de contracter un cancer, et
ledit cancer étant un cancer du sein et,
ladite pro-neurotensine 1-117 étant un peptide selon SEQ ID NO 5, et
ladite pro-neurotensine 1-117 comprenant des peptides étant choisie dans un groupe de peptides comprenant les séquences de peptide de séquences SEQ ID NO 1, 2, 6 et 7, et
le liquide corporel étant prélevé sur un sujet de sexe féminin à jeun.

2. Méthode selon la revendication 1, le taux élevé signifiant un taux au-dessus d'un niveau seuil.

3. Méthode selon une quelconque des revendications 1 ou 2, ledit sujet de sexe féminin n'ayant pas d'antécédents de diagnostic de cancer au moment où l'échantillon du fluide corporel est prélevé dudit sujet de sexe féminin.

4. Méthode selon une quelconque des revendications 1 à 3, ledit sujet de sexe féminin ayant eu des antécédents de diagnostic de cancer et ayant été guéri au moment où l'échantillon du fluide corporel est prélevé dudit sujet de sexe féminin et le risque de réapparition du cancer du sein étant déterminé.

5. Méthode selon une quelconque des revendications 1 à 4, une maladie cardiovasculaire ou un diabète ayant été diagnostiqué chez ledit sujet de sexe féminin au moment du prélèvement du fluide corporel.

6. Méthode selon la revendication 5, ladite maladie cardiovasculaire pouvant être choisie parmi le groupe comprenant l'accident vasculaire, l'athérosclérose et l'hypertension, au moment où l'échantillon du fluide corporel est prélevé dudit sujet de sexe féminin

7. Méthode selon la revendication 5, un diabète de type II ayant été diagnostiqué chez ledit sujet de sexe féminin au moment du prélèvement du fluide corporel.

8. Méthode selon une quelconque des revendications 1 à 7, en outre au moins un paramètre clinique déterminé étant choisi parmi le groupe, comprenant : âge, présence du diabète sucré et tabagisme actuel.

9. Méthode selon l'une quelconque des revendications précédentes, permettant de déterminer le taux de pro-neurotensine 1-117.

10. Méthode selon l'une quelconque des revendications précédentes, le taux de pro-neurotensine 1-117 ou de ses fragments ou de pro-neurotensine 1-117 comprenant des peptides étant mesuré à l'aide d'un immunoessai.

11. Méthode selon l'une quelconque des revendications 1 à 10, ladite méthode étant effectuée plus d'une fois pour surveiller le risque de contracter le cancer du sein chez un sujet de sexe féminin.

12. Méthode selon la revendication 11, ladite surveillance étant réalisée pour évaluer la réponse dudit sujet de sexe féminin à des mesures préventives et/ou thérapeutiques prises.

13. Méthode selon une quelconque des revendications 1 à 12 pour stratifier lesdits sujets de sexe féminin en groupes de risque.

14. Méthode selon la revendication 13, ledit sujet de sexe féminin étant reclassé à cause de la détermination du taux de pro-neurotensine 1-117 ou de ses fragments d'au moins 5 acides aminés ou de pro-neurotensine 1-117 comprenant des peptides, dans un liquide corporel provenant dudit sujet de sexe féminin.
